# EUROPEAN PATENT APPLICATION

(11) **EP 4 635 536 A1**
(43) Date of publication of application: **22.10.2025**
(21) Application number: 24382408.3
(22) Date of filing: 17.04.2024
(51) Int. Cl.: A61M 5/34, A61M 5/32

(54) **PUNCTURE EXTENSION DEVICE**

(71) Applicant: Clinicas Dr. Juan Cabrera, S.L., 18005 Granada (ES)
(72) Inventor: CABRERA GARRIDO, Juan, E-18005 Granada (ES); LÓPEZ GONZÁLEZ, Gonzalo, E-15009 A Coruña (ES)
(74) Representative: ABG Intellectual Property Law, S.L.

(57) **Abstract**

The invention refers to an extension device (10) for a puncture device (20) to puncture structures located within an anatomical cavity, where the extension device comprises a hollow tube (1) with a distal end (2) and a proximal end (3); where the distal end (2) comprises a luer cone connector adapted for coupling to a hypodermic needle (4); and the proximal end (3) comprises a threaded luer connection adapted for coupling to a source of sclerosing substance.

Likewise, the invention also includes a puncture device (20) and an injection, drainage, and/or sampling system (30) that incorporates this puncture device (20).

## Description

### FIELD OF THE INVENTION

The present invention is directed to a puncture extension device apt for puncturing/injecting structures within anatomical cavities, e.g., for outpatient sclerotherapy treatments. It is also directed to a puncture device that includes the puncture extension device and to a system that includes the puncture device.

### BACKGROUND OF THE INVENTION

Hemorrhoidal disease most frequently develops between the ages of 45 and 65 years, with no differences between the sexes. Its prevalence among over-50-year-olds was found to be around 50%. It is diagnosed in around 39% of colorectal cancer screening colonoscopies. The etiopathogenesis of hemorrhoid disease has not been fully elucidated, but it has been associated with factors that increase pressures on rectal venous plexuses, including constipation, obesity, pregnancy, chronic diarrhea, or excessive time (>3-5 min) on the toilet. The most frequent symptoms/signs are anal bleeding (the most common), pruritus, pain (spontaneous or during defecation), prolapse, mucus secretion, and fecal incontinence, among others. The treatment of hemorrhoidal disease depends on the severity of signs and symptoms and the examination findings (evaluation of Goligher grade).

An increase in dietary fiber intake and hydration, a reduction in defecation time, and the utilization of Sitz baths have been proposed as useful conservative measures at initial stages of the disease. Other strategies to relieve symptoms include local topical treatments with corticosteroids, anesthetics, vasoconstrictors, or emollients, among others, although no studies have been published on their long-term effectiveness or potential side effects.

A more aggressive approach is adopted at advanced stages of the disease (Goligher grade III or IV) or in the absence of a response to conservative treatment. Aggressive treatments include hemorrhoidectomy, stapled hemorrhoidopexy, dearterialization, or a heat-based procedure with radiofrequency or laser fibers. These treatments involve dramatic aggression of the area, with notable differences in efficacy, recurrences, and complications: early, such as postoperative pain (common to all), anal bleeding, urinary retention, and plexus thrombosis, which can be very severe, producing perianal abscess, massive bleeding, sepsis, or peritonitis; or late, such as anal stenosis, skin tags, or fecal incontinence.

Ligation techniques include the placing of rubber bands at the base of internal hemorrhoids (Goligher grades I-III) through endoscopy or a single-use device. These techniques are equally aggressive because they produce, by ischemia, the necrosis and subsequent detachment of the ligated pedicles. One limitation is that spinal or epidural anesthesia is usually needed to treat multiple hemorrhoids in a single act.

Treatment based on thermocoagulation applies heat sources (e.g., laser or radiofrequency) to the base of hemorrhoidal pedicles to thrombose afferent arteries and produce fibrosis. Some examples of these thermocoagulation techniques are reported by COCORULLO et al. in "The non-surgical management for hemorrhoidal disease. A systematic review", II Giornale di chirurgia, 2017, vol. 38. This approach has a four-fold lower recurrence rate in comparison to rubber band ligation but is more uncomfortable for patients, and the recovery is slower. For its part, cryotherapy has been relegated to the topical application of local cold for pain relief, due to its low effectiveness and important side effects, including prolonged secretion at the treated area.

Among attempts to develop minimally aggressive but effective treatments, sclerotherapy has been used to induce inflammatory fibrosis. Although of little benefit when the sclerosant is administered as a liquid, it is highly effective when delivered as an anionic foam. The success of this radical advance has been described by Fernandes, V., & Fonseca, J. in "Polidocanol foam injected at high doses with intravenous needle: the (almost) perfect treatment of symptomatic internal hemorrhoids". GE-Portuguese Journal of Gastroenterology, 26(3), 169-175, 2019.

High concentrations (around 3%) of polidocanol have been used for the sclerosing treatment of hemorrhoids, as reported by LOBASCIO et al. in "Short-term results of sclerotherapy with 3% polidocanol foam for symptomatic second- and third-degree hemorrhoidal disease". Journal of Investigative Surgery, 2021, vol. 34, no 10, p. 1059-1065.

In the treatment of hemorrhoids by minimally invasive sclerotherapy, different systems can be employed to access the affected area (anoscope, proctoscope, fiberscope, etc.), and various substances (with different concentrations) and needle types (with different diameters and lengths) can be used. The most widely used substances are polidocanol and sodium tetradecyl sulfate, as reported by Benin, P., & D'Amico, C. (2007) in "Foam sclerotherapy with Fibrovein (STD) for the treatment of hemorrhoids, using a flexible endoscope", Minerva chirurgica, 62(4), 235-240, 2007). They are both anionic surfactants that allow foam to be obtained in the treatment center. These surfactants are used at concentrations between 2 and 3% with 20-23 G needles that are more than 12 mm in length. Despite achieving good outcomes, reported complications include pain from the puncture (sometimes requiring anesthesia), inflammatory reactions, and severe adverse effects such as bowel perforation, prostatic injection, and rectovesical fistula. Foam sclerotherapy for hemorrhoid disease is most frequently used for Goligher grades I and II.

Access to hemorrhoidal cushions and their treatment are hampered by their anatomical localization in the rectal ampulla distal to the dentate line at 20-30 mm from the anal orifice, depending on the individual. In addition, the wall of the rectal ampulla, which is adjacent to other structures (e.g., vagina, urethra, prostate, and peritoneum) is thin and readily perforated.

In sclerotherapy treatments, anoscopes are frequently used to visualize the anal cavity and access the hemorrhoid(s). The shape and dimensions of anoscopes (90-103 mm in length and 18-23 mm in diameter) means that they offer a limited monocular field of view. Furthermore, if an additional element (e.g., puncture device) is introduced for hemorrhoid treatment, the clinician's field of view is even more limited.

These drawbacks have conventionally been overcome by using thick and long needles (typically spinal anesthesia needles >23 G in diameter and 150 mm in length), but this makes it more difficult to achieve a precise puncture at a specific site and depth. Their utilization worsens the discomfort of patients (e.g., pain and/or profuse bleeding) and especially increases the risk of bowel perforation, potentially involving surrounding organs, forming fistulae (e.g., intestinal-prostatic, vesical, vaginal), or even resulting in abdominal sepsis.

This problem affects not only the sclerotherapy treatment of hemorrhoids but also other procedures requiring the puncture of structures that cannot be readily accessed due to their localization within an anatomical cavity.

However, no puncture device is available that provides clinicians with a wide field of view of the intervened area (e.g., hemorrhoidal cushion for sclerotherapy) and allows them to perform a safe and effective puncture with a short, small-gauge needle.

Hence, there is a need for the development of novel devices that improve the clinician's visualization of anatomical cavities to be punctured, e.g., for the outpatient treatment of hemorrhoid disease by sclerotherapy. These puncture devices also need to be compatible with the anoscopes conventionally available to clinicians.

### DESCRIPTION OF THE INVENTION

This invention proposes to resolve the above-reported problems by using a puncture extension device according to claim 1, a puncture device according to claim 7, and an injection, drainage, and/or sampling system according to claim 10. The dependent claims define the performances of the invention.

The present invention solves the above-reported problems by providing the clinician with a wider view while being compatible with conventional anoscopes. Likewise, the present invention permits a less invasive puncture of structures within anatomical cavities, minimizing discomfort and health risks for patients and avoiding the involvement of surrounding anatomical regions.

A first inventive aspect is a puncture extension device for the puncture of structures within an anatomical cavity. It comprises a hollow tube with distal and proximal ends where:
- The distal end has a luer cone connector adapted for a hypodermic needle; and
- The proximal end has a threaded luer lock connection.

In the context of the invention, the anatomical cavity is understood to be not only a space accessible through a bodily orifice (e.g., mouth, oropharynx, anus, or vagina) but also a space opened by an incision in the body (e.g., chest, abdomen, or skull).

In the context of the invention, a "structure" is a solid or cystic tissue mass (tumor) of any origin (vascular, dysplastic, hypertrophic, or neoplastic). Examples of structures in terms of the invention are: hemorrhoid, angioma, varicose vein, abscess, cyst, and tumor. In this document, the terms "structure" and "anatomical structure" are used indistinguishably.

For the purpose of abbreviation, the "puncture extension device" is referred to as the "extension device".

The "distal end" is the end closest to the anatomical structure to be punctured using the device. The "proximal end" is the end closest to the clinician using the device.

In the field of the invention, a threaded luer connection refers to the connection described in norm ISO 80369.

In a preferred performance, the means of threaded luer connection of the proximal end include a luer lock connection. Advantageously, the luer lock connection also has a threaded lock that guarantees a hermetic and extraction-resistant connection.

In other performances, the means of threaded luer connection of the proximal end include a female luer connection.

Thanks to the luer cone shape of its distal end, the extension device of the present invention can connect to a short (e.g., 12 mm), small-gauge (e.g., 27-30 G) needle for the precise and safe puncture of an anatomical structure (hemorrhoid or tumor). In the proximal end, the device of the invention includes means of threaded luer connection, configured to stably connect the device to an additional element, such as source of sclerosant substance, syringe, or aspiration device.

In the field of needles for medical application, the gauge of needles is designated G. Higher gauge numbers indicate thinner needles. G refers to the thickness (diameter) of the needle, e.g., a 23 G needle has a diameter of 0.6 mm, while a 25 G needle has a diameter of 0.5 mm.

In the context of the present invention, "short" needles are those with a length of between 5 and 12 mm, preferably between 5 and 10 mm. Likewise, in the field of this invention, "small gauge" needles are those with a diameter between 27 and 30 G (between 0.41 and 0.3 mm). Small gauge needles in the present invention have objectively smaller dimensions than those habitually used for hemorrhoid puncture.

In a preferred performance, the device of the present invention is applied for hemorrhoid puncture. However, this use is nonrestrictive, and the device can be used for other diseases and/or procedures, including the injection of a drug in an anatomical structure, drainage of an anatomical structure, or sampling from an anatomical structure.

For applications in the field of sclerotherapy, the device of the invention allows clinicians to reach hemorrhoids through an anoscope and inject them in a precise and safe manner by only minimally limiting the field of view of the anoscope and therefore of the area to be treated. Likewise, the device of the invention can also be used to reach and sclerotize an anatomical structure in another cavity (e.g., mouth) to reach an anatomical structure in which a drug must be injected, to reach an anatomical structure to be drained, and/or to reach an anatomical structure to be biopsied. In all applications, the anatomical structure to be punctured can be within an anatomical cavity accessed through a natural opening (e.g., mouth, anus) or in an anatomical cavity accessed through an incision (e.g., trunk), facilitating a minimally invasive incision in comparison to the introduction, at least partially, of the hand of the clinician.

In preferred performances, the source of sclerosing substance is a syringe, vial, or container filled with a sclerosant substance to be applied on the hemorrhoid or other anatomical structure.

In a performance, a central portion of the hollow tube is cylindrical.

In a performance, the hollow tube is rigid.

In the field of the present invention, a "rigid" tube is a tube that does not fold or bend from its own weight and that of a needle connected to it, allowing punctures to be made with the distal end while it is held by the proximal end. One example of material to fabricate this hollow tube is photopolymer Somos ^{®} ProtoGen 18420, which provides the necessary rigidity. However, other materials can be used to fabricate the hollow tube.

Advantageously, the rigidity of the material used to fabricate the device of the present invention results in its easy handling by the clinician.

In a performance, the hollow tube is made with one or more of the following materials:
- polycarbonate,
- polypropylene,
- polyethylene,
- vinyl polychloride,
- acrylonitrile butadiene styrene,
- polyamide,
- polyetheretherketone, or
- polyethylene terephthalate.

In a performance, the hollow tube has a length of between 100 and 260 mm, preferably between 130 and 250 mm, and more preferably between 230 and 240 mm. Advantageously, these dimensions favor handling of the device (and of a puncture device of which the device is part) by the clinician and at the same time enhance the visibility of the puncture device during the intervention, avoiding interference with the view by the hand of the clinician who is handling it. Hence, the device (and therefore the puncture device) allows the affected area to be reached without hampering the view of the operator.

In a performance, the central portion of the hollow tube has an outer diameter of between 3 and 9 mm, preferably between 5 and 7 mm, and more preferably between 5.5 and 6.5 mm.

Advantageously, thanks to its small gauge (it only occupies between 25-30% of the lumen of a conventional anoscope), the extension device of the present performance permits reaching the intervened area without the view being obstructed by the hand of the operator.

In a performance, the hollow tube has an inner diameter between 2.5 and 3.5 mm, preferably between 2.8 and 3.2 mm.

In a second inventive aspect, the invention provides a puncture device to puncture structures located within anatomical cavities, where the puncture device comprises:
- an extension device according to any of the performances of the first inventive aspect, and
- a hypodermic needle coupled to the connector.

In a performance of the puncture device, the hypodermic needle has a length <12 mm, preferably <10 mm.

In a performance of the puncture device, the hypodermic needle has a gauge between 25 and 30 G, more preferably between 27 and 30 G.

In a preferred performance, the puncture device of the present invention is a hemorrhoid puncture device. However, this use is not restrictive, and the puncture device can be applied in other diseases and/or procedures.

In a third inventive aspect, the invention provides an injection, drainage, and/or sampling system that comprises:
- a puncture device according to any of the performances of the second inventive aspect, and
- a source of sclerosing substance, a syringe, or aspiration device, with this source of sclerosing substance, syringe, or aspiration device being connected to the proximal end of the extension device.

In a performance, the system comprises a source of sclerosing substance connected to the proximal end of the extension device. In this performance, it is an injection system configured to inject a sclerosing substance in an anatomical structure, such as a hemorrhoid.

In a performance, the source of sclerosing substance is a syringe, vial, or container filled with the sclerosing substance to be applied in the hemorrhoid or other anatomical structure.

In a performance, the source of sclerosing substance contains one or more of the following sclerosing substances: polidocanol, sodium tetradecyl sulfate, hypertonic glucose or glucosaline solution, chromic glycerol, ethanolamine oleate, sodium morrhuate, iodine solution, or 5% phenol in almond oil. In preferred performances, sclerosing substances can be liquid or microfoam.

In a performance, the sclerosing substance comprises a polidocanol concentration between 0.37 and 1%, more preferably between 0.37 and 0.5%, and even more preferably 0.5 %.

In a performance, the sclerosing substance is a medical-use injectable microfoam. In a performance, the microfoam includes any of the aforementioned sclerosing substances (e.g., polidocanol, sodium tetradecyl sulfate, etc.).

In a performance, the source of sclerosing substance contains a volume of between 5 and 15 mm of the sclerosing substance.

In a performance, the system comprises a syringe connected to the proximal end of the extension device. In this performance, the system is an injection, drainage, and/or sampling system. In a performance, the syringe includes a drug or other substance to be injected in an anatomical structure, such as a sclerosing substance. In a performance, the syringe is configured to drain a substance from the interior of an anatomical structure and/or for sampling from the anatomical structure, for instance an abscess, cyst, or tumor.

In a performance, the system comprises an aspiration device connected to the proximal end of the extension device. In this performance, the system is a drainage and/or sampling system and the aspiration device is configured to drain a substance from the interior of an anatomical structure and/or to take a sample from the anatomical structure (e.g., an abscess, cyst, or tumor).

In a performance, the system comprises an anoscope, preferably with a diameter between 15 and 23 mm, and more preferably a diameter of 18 mm.

A fourth aspect of the invention refers to a method of hemorrhoid treatment by sclerotherapy, which comprises the following steps:
- apply an anoscope (preferably with a small diameter, more preferably around 15-20 mm) to the surroundings of an area with at least one hemorrhoidal cushion;
- puncture at least one hemorrhoidal cushion using the needle of the extension device of the present invention; and
- employ the puncture device of the present invention to apply a sclerosing substance, preferably polidocanol microfoam, in at least one hemorrhoidal cushion for its sclerotization.

In a performance, the application of the sclerosing substance is performed using a source of sclerosing substance connected to the proximal end of the extension device.

An additional aspect of the invention refers to a method for drug injection in an anatomical structure, which comprises the following steps:
- puncture the anatomical structure using the needle of the extension device of the present invention; and
- inject the drug into the anatomical structure using the puncture device of the present invention.

In a performance, the drug is injected with a syringe connected to the proximal end of the extension device.

An additional aspect of the invention refers to an anatomical structure drainage method, which comprises the following steps:
- puncture this anatomical structure using the needle of extension device of the present invention; and
- drain the anatomical structure using the extension device of the present invention.

In a performance, the anatomical structure is drained using a syringe or aspiration device connected to the proximal end of the extension device.

An additional aspect of the invention refers to an anatomical structure sampling method, which comprises the following steps:
- puncture the anatomical structure using the needle of the extension device of the present invention; and
- take a sample from the anatomical structure using the extension device of the present invention.

In a performance, samples are taken from the anatomical structure using a syringe or aspiration device connected to the proximal end of the extension device.

All characteristics of the invention described in the present document (including claims, description, and figures) can be combined in any manner, except for combinations with mutually excluding characteristics.

### FIGURE DESCRIPTION

These and other characteristics and advantages of the invention are more clearly demonstrated in the detailed description of a preferred form of performance, provided solely as an illustrative and nonrestrictive example, with reference to the accompanying figures.
- Figure 1: This figure depicts a lateral view of an extension device performance in accordance with the present invention.
- Figure 2: This figure depicts a cross-sectional view of the A-A' dimension in Figure 1.
- Figure 3: This figure depicts a perspective view of the extension device in accordance with the present invention.
- Figure 4: This figure depicts a detailed cross-sectional view of the ends of a performance of the extension device in accordance with the present invention.
- Figure 5: This figure depicts a detailed view of the distal end of a performance of the extension device of the invention in two different configurations: a) before its connection to the hypodermic needle, and b) after its connection to the hypodermic needle.
- Figure 6: This figure depicts a view of the injection system in accordance with a performance of the present invention, comprising the extension device and an anoscope.

The above figures use numerical references gathered in the following table.

**Numerical reference index**

| | |
|---|---|
| 1 | Hollow tube |
| 1.1 | Central portion of the hollow tube |
| 2 | Distal end |
| 3 | Proximal end |
| 4 | Needle |
| 10 | Puncture extension device |
| 15 | Anoscope |
| 20 | Puncture device |
| 30 | Injection system |
| L1 | Length of the hollow tube |
| L2 | Length of the distal end |
| L3 | Length of a portion of the proximal end |
| L4 | Length of the proximal end |
| D1 | Outer diameter of the central portion of the hollow tube |
| D2 | Minimum value of the inner diameter (lumen) of the distal end |
| D3 | Thickness of the central portion of the hollow tube |
| D4 | Inner diameter (lumen) of the central portion of the hollow tube |
| D5 | Maximum value of the outer diameter of the proximal end |

### DETAILED DESCRIPTION OF THE INVENTION

The attached images, which are included in and form part of this specification, illustrate the performances of the invention and, alongside the description, serve to explain the principles of the extension device (10) of the invention. These performances must be interpreted in an illustrative manner, nonrestrictive of possible preferred implementations of the invention.

Although the extension device performances described below (10) are for hemorrhoid puncture, this functionality is not exclusive. The extension device of the invention may equally be used to reach, sclerotize, drain, or biopsy an anatomical structure that is within an orifice (e.g., the mouth) or is accessed through an incision (e.g., in the trunk), making this incision minimally invasive in comparison to the at least partial introduction of the clinician's hand.

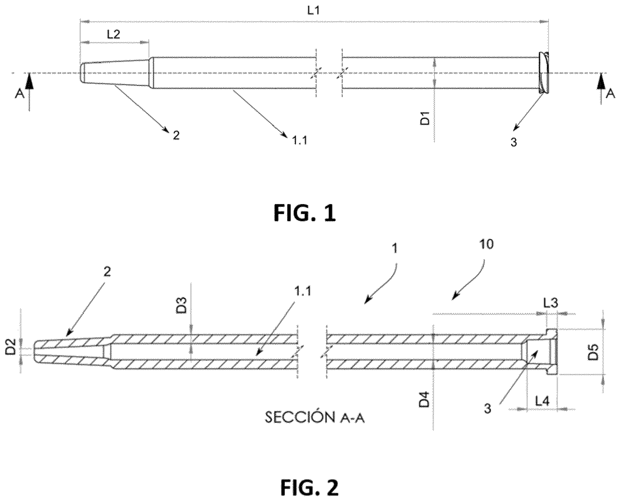

Figure 1 depicts a lateral view of a performance of the extension device (10) of the present invention, which comprises a hollow tube (1) of given length (L1) that includes a distal (2) and a proximal (3) end. In a performance, the hollow tube (1) is rigid.

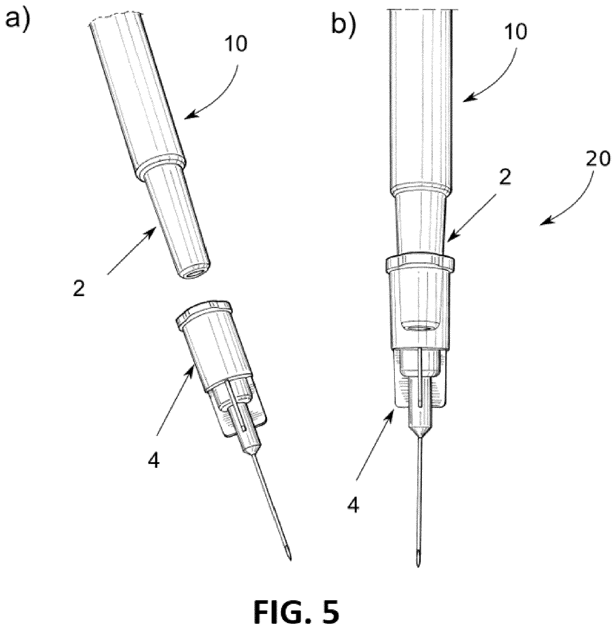

The distal end (2) of the extension device (10) comprises a luer cone connector adapted for coupling to a needle (4) such as a hypodermic needle, not shown in Figure 1 but illustrated in Figure 5, being the needle used to puncture the anatomical structure (e.g., hemorrhoidal cushion).

The proximal end (3) of the extension device (10) comprises a threaded luer-lock connection adapted for stable connection to an additional device, such as a source of sclerosing substance (not shown in the Figure) that contains a sclerosing substance to treat the hemorrhoidal cushion. In other performances, the threaded luer-lock connection is used for connecting to a syringe for draining and/or sampling from the anatomical structure or to a syringe for injecting a drug in the anatomical structure, or for connecting to an aspiration device for draining and/or sampling from the anatomical structure.

In a performance, the central portion (1.1) of the hollow tube (1) is cylindrical.

In a performance, the central portion (1.1) of the hollow tube (1) extends from the threaded luer cone connector. In this performance, the central portion (1.1) encompasses the whole hollow tube (1) except for the distal end (2) and the proximal end (3).

Figure 2 depicts a cross-sectional view of the extension device (10) in Figure 1. In a performance of the extension device (10), the hollow tube (L1) has a length between 137 and 247 mm, an outer diameter (D1) of the central portion (1.1) between 5.84 and 6.84 mm, and a lumen or inner diameter (D4) of the central portion (1.1) between 2.80 and 3.20 mm. Likewise, in this performance, the length of the portion (L3) of the proximal end (3) ranges between 0.5 and 1.8 mm.

In other performances, the length (L1) of the hollow tube (1) ranges between 100 and 260 mm, more preferably between 150 and 250 mm. Advantageously, the fact that the hollow tube length is at least 100 mm means that the hand of the clinician does not interfere with the field of view through the anoscope (15).

In some performances, one or various dimensions of the extension device range between the following measurements:
- L2 between 10 and 16 mm,
- D2 between 0.5 and 1.5 mm,
- D3 (width of the central portion [1.1]) between 1 and 2 mm,
- D5 between 6.7 and 8.7 mm and/or
- L4 between 4.1 and 6.1 mm.

As observed in the performance of Figure 2, the inner diameter (lumen) of the distal end (2) gradually varies, such that D2 denotes its minimum value. The value of D5 denotes the maximum value of the outer diameter of the proximal end (3).

In a performance, the dimensions of the extension device (10) are (all indicated dimensions have an error of ±0.1 mm): L1= 230.2 mm, L2= 13.0 mm, D1= 5.8 mm, D2= 1.0 mm, D3= 1.5 mm, D4= 2.8 mm, D5= 7.7 mm, L3= 1.8 mm, and L4= 5.1 mm.

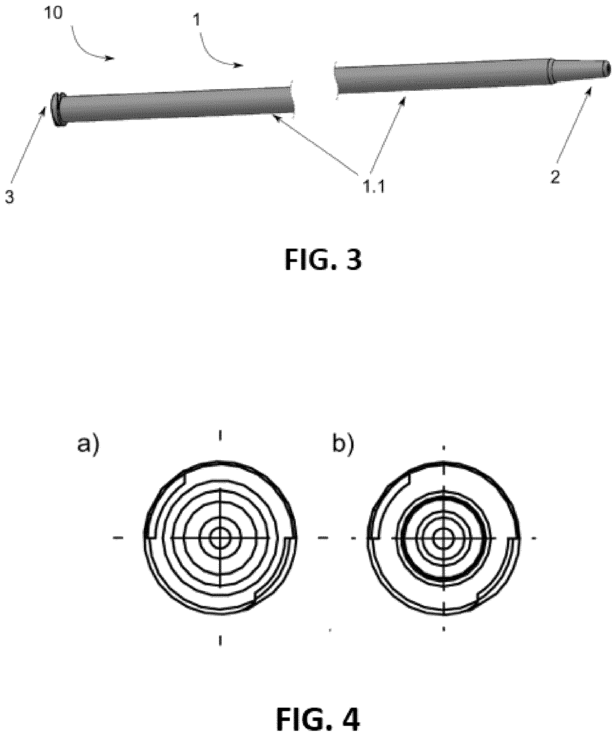

Figure 3 depicts a perspective view of the extension device (10) in Figures 1-2. Given that the length of the hollow tube (L1) is much higher in comparison to the other dimensions of the device, the hollow tube (1) is represented by a broken line. In certain performances, the length of the hollow tube (L1) ranges between 137 and 247 mm.

The extension device (10) of the invention is particularly advantageous due its great versatility, permitting not only application of the sclerosing substance from a source connected to the proximal end (3) but also puncture of the hemorrhoidal cushion with a needle (4) connected to the distal end (2). In addition, the connections of the ends (2, 3) to the source of sclerosing substance and needle (4), respectively, allow the hygienic reutilization of the extension device (10), whereas hypodermic needles (4) are discarded after their use. In addition, the sources of sclerosing substances can be reutilized or, alternatively, discarded after their use.

In preferred performances, the sclerosing substance is a foam, preferably a polidocanol microfoam at low concentrations (between 0.37 and 1%). In particular, the microfoam is applied with a source of sclerosing substance connected to the proximal end (3) of the extension device (10).

The application of a polidocanol microfoam at low concentrations (between 0.37 and 1%) is especially advantageous because, being lower concentrations of the active ingredient in comparison to those habitually used (around 3%), there is a smaller possibility of potential adverse effects such as inflammations, erosion, tissue ulceration, etc.

Figure 4 depicts detailed views of the cross-section of a performance of the extension device (10). Specifically, Figure 4A depicts the detail viewed from the distal end (2) for the coupling of a needle (4) (the end that includes a luer cone connector), and Figure 4B depicts the detail from the proximal end (3), which comprises the luer lock connection. This luer lock connection in the invention provides stability in the union with the source of sclerosing substance or with another device. However, in other performances of the invention, the connection is a female luer connection.

Advantageously, the luer lock connection in the proximal end (3) allows the source of the sclerosing solution to be securely blocked and avoids leaks of the solution. In addition, the luer lock connection allows the injection to be performed in a precise and controlled manner. The stability afforded by the luer lock connection is superior to that obtained with the luer connection.

The extension device (10) can be fabricated, for example, by 3D printing with laser stereolithography (SLA) using a liquid photopolymer such as ProtoGen O-XT 18420.

A medical-use plastic material can be utilized for the large-scale manufacture of the extension device (10) of the invention by employing molds. This material should preferably be one of the following: polycarbonate (PC), polypropylene (PP), polyethylene (PE), polyvinyl chloride (PVC), acrylonitrile butadiene styrene (ABS), polyamide (PA), polyetheretherketone (PEEK), or polyethylene terephthalate (PET). In some performances, two or more of the above plastic materials can be combined. Advantageously, the use of these plastic materials offers great versatility to fabricate the extension device (10) of the invention.

When the plastic material is PE, both high-density PE (HDPE) and low-density PE (LDPE) are suitable to fabricate the extension device (10).

Figure 5 illustrates a detailed view of the distal end (2) of the extension device (10) of the invention. Figure 5a) depicts this extension device (10) decoupled from a hypodermic needle (4) and clearly illustrates the luer cone at the distal end (2), while Figure 5b) depicts the distal end after coupling the needle (4), configuring a puncture device (20) according to the invention.

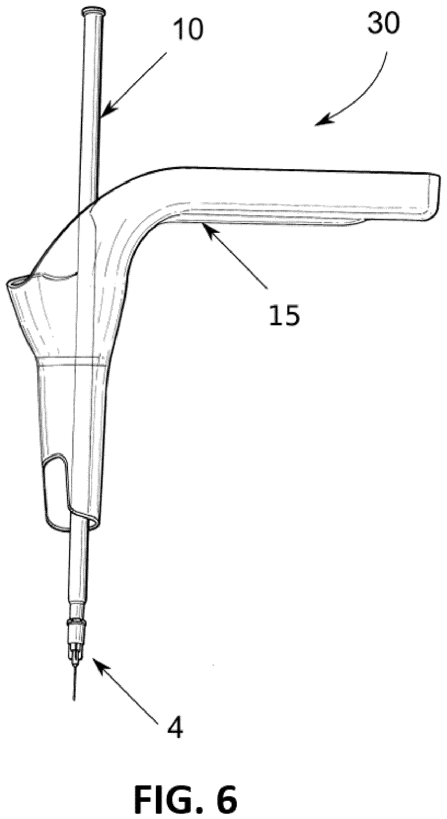

Figure 6 depicts an injection system (30) according to a performance of the invention, which comprises the puncture device (20) of the invention alongside an anoscope (15). As observed, the puncture device (20) is readily inserted into the lumen of the anoscope, which has a much larger diameter than that of the puncture device (20). In a performance, the anoscope (15) can be self-illuminating to facilitate examination of the area to be treated. The use of a small anoscope (e.g., 15-20 mm in diameter) alongside the puncture device (20) of the invention permits effective access to hemorrhoids and their safe puncture with needles <12 mm in length with a gauge between 25 and 30 G.

### Experimental outcomes

The puncture device (20) of the present invention has been tested in 700 patients with hemorrhoidal disease.

The treatment is based on the utilization of this puncture device (20) to selectively puncture the hemorrhoidal cushion. This puncture device (20) is used in combination with a self-illuminating disposable anoscope (15) that provides a monocular view and usually limits the three-dimensional field of view of the clinician. However, an improvement is achieved in this restricted field of view due to the small diameter of the puncture device (20) of the present invention, allowing clinicians to perform safer injections of the treatment in the selected site using thin needles (e.g., 27 G) of minimal length (e.g., 12 mm).

Patient preparation requires a small saline enema a few hours before the treatment. Puncture with thin needles is painless and perceived as mild discomfort. Alongside its simplicity and rapidity, this results in a well-tolerated procedure and allows patients to leave the consulting room without further delay.

The sclerotherapy technique for hemorrhoid treatment according to the present invention includes the following steps:
- utilization of an anoscope (15) (preferably of small diameter, around 15-20 mm, e.g., 18 mm) to examine the surroundings of an area with at least one hemorrhoidal cushion,
- puncture of at least one hemorrhoidal cushion using a hypodermic needle (4) of the puncture device (20) of the present invention, and
- application of polidocanol microfoam in at least one hemorrhoidal cushion using the puncture device (20) of the present invention.

In the tested treatment, the polidocanol concentration in the microfoam used was between 0.37 and 1% (0.5% in 86% of cases), and the volume injected was between 5 and 15 cubic centimeters (cc) at each session.

These concentrations of polidocanol in the microfoam are particularly advantageous in terms of effectiveness (good outcomes with minimal likelihood of adverse effects). We highlight that the polidocanol concentration range is significantly lower than the concentration habitually used, which is most frequently 3%.

Advantageously, the above technique facilitates the precise and safe puncture of hemorrhoidal cushions and achieved the control of associated symptoms in almost all patients (around 95%) after a small number of outpatient sessions (median=1, range 1-4) of short duration (sessions of between 5 and 15 min, average of 10 min), with scant cases of discomfort or adverse effects.

Although the sclerotherapy technique has been described using polidocanol microfoam as sclerosing substance, this technique can be applied using any other sclerosing substance.

In summary, the extension device (10) and the puncture device (20) of the invention effectively allow access to hemorrhoidal cushions and their safe puncture using thin (e.g., 27 G) and short (e.g., 12 mm) needles.

## Claims

1. Extension device (10) for puncture device to puncture structures within an anatomical cavity, where the extension device (10) comprises a hollow tube (1) with a distal end (2) and a proximal end (3); where:
- the distal end (2) comprises a luer cone connector adapted for coupling to a hypodermic needle (4); and
- the proximal end (3) comprises a threaded luer connection.

2. Extension device (10) for puncture device according to claim 1, where:
- a central portion (1.1) of the hollow tube (1) is cylindrical; and/or
- the hollow tube (1) is rigid.

3. Extension device (10) or puncture device according to any of the above claims, where the hollow tube (1) is manufactured in one or more of the following materials:
- polycarbonate,
- polypropylene,
- polyethylene,
- polyvinyl chloride,
- acrylonitrile butadiene styrene,
- polyamide,
- polyetheretherketone, or
- polyethylene terephthalate.

4. Extension device (10) for puncture device according to any of the above claims, where which the hollow tube (1) has a length of between 100 and 260 mm, preferably between 130 and 250 mm, and more preferably between 230 and 240 mm.

5. Puncture extension device (10) according to any of the above claims, where the hollow tube (1) has an outer diameter between 3 and 9 mm, preferably between 5 and 7 mm, and more preferably between 5.5 and 6.5 mm.

6. Puncture extension device (10) according to any of the above claims, where the hollow tube (1) has an inner diameter between 2.5 and 3.5 mm, preferably between 2.8 and 3.2 mm.

7. Puncture device (20) for the puncture of structures within an anatomical cavity, where the puncture device (20) comprises:
- an extension device (10) according to any of the above claims, and
- a hypodermic needle (4) coupled to the connector.

8. Puncture device (20) according to the above claim, where the hypodermic needle (4) has a length <12 mm, preferably <10 mm.

9. Puncture device (20) according to claims 7-8, where the hypodermic needle (4) has a gauge between 25 and 30 G, more preferably between 27 and 30 G.

10. Injection, drainage, and/or sampling system, which includes:
- a puncture device (20) according to claims 7-9, and
- a source of sclerosing substance, a syringe, or an aspiration device, with this source being connected to the proximal end (3) of the extension device (10).

11. System according to the above claim, where the source of sclerosing substance includes one or more of the following sclerosing substances: polidocanol, sodium tetradecyl sulfate, glucose or glucosaline hypertonic solution, chromic glycerol, ethanolamine oleate, sodium morrhuate, iodine solution, or 5% phenol in almond oil.

12. System according to the above claim, where the sclerosing substance comprises a polidocanol concentration between 0.37 and 1%.

13. System according to claims 10-12, where the sclerosing substance is a microfoam.

14. System according to claims 10-13, where the source of sclerosing substance contains a volume between 5 and 15 mm of sclerosing substance.

15. System according to claims 10-14, which additionally comprises an anoscope (15), preferably with a diameter between 15 and 20 mm, and more preferably 18 mm.
